# EUROPEAN PATENT SPECIFICATION

(11) **EP 2 227 187 B1**
(45) Date of publication and mention of the grant of the patent: **12.06.2019**
(21) Application number: 08853249.4
(22) Date of filing: 24.11.2008
(51) Int. Cl.: A61F 2/95

(54) **DOUBLE LOADED STENT DELIVERY SYSTEM**
DOPPELT BELADENES STENTABLAGESYSTEM
SYSTÈME D'ADMINISTRATION DE DOUBLE STENT CHARGÉ

(30) Priority: 28.11.2007 US 946469
(43) Date of publication of application: 15.09.2010
(73) Proprietor: Cook Medical Technologies LLC, Bloomington, IN 47404 (US)
(72) Inventor: LU, Wenfeng, Pfafftown NC 27040 (US); ZHANG, Cuixin, Pfafftown NC 27040 (US)
(74) Representative: Garratt, Peter Douglas
(86) International application number: PCT/US2008/084494
(87) International publication number: WO 2009/070523

(56) References cited:
- WO-A-2006/005082
- WO-A2-01/82831
- US-A- 5 064 435
- US-A1- 2001 044 650
- US-A1- 2007 118 202

## Description

### BACKGROUND

The present invention relates generally to medical devices and more particularly to stents and a system for delivering the stents to dilate narrowed portions of a lumen.

Stents are widely used in the medical profession for various intraluminal procedures. One common procedure involving the use of a stent relates to dilation of a stenosed region, which is a narrowing of a body lumen. For example, strictures or occlusions that develop in the upper common bile duct and/or the left and right hepatic ducts can interfere with the proper drainage of those ducts. Accordingly, biliary stents are often used to maintain the patency of the biliary tree or common bile duct.

Self-expanding metallic stents having a polymeric covering are widely used today. The metallic stent provides sufficient radial force against the stenosed region and the polymeric covering disposed along the metallic stent prevents cancerous tissue from growing into the interstices of the metallic stent. Accordingly, covered metallic stents are widely favored in many areas of the body. For example, it may be advantageous to use covered metallic stents in the biliary, duodenal, colonic regions.

The delivery of such covered metallic stents is achieved with a conventional introducer, which includes an outer sheath coaxially disposed over an inner sheath. Proximal retraction of the outer sheath relative to the inner sheath enables the covered metallic stent to self-expand against the stricture.

Limited introducer space may prevent delivery of a metallic stent having a covering. The limited introducer space may be due to the relatively small diameter of the working channel of an endoscope or introducer through which the introducer must be inserted during delivery. As an example, the working channel of a typical therapeutic duodenoscope cannot exceed about 4.2 mm in diameter. The largest introducer which can be inserted into such a working channel is about 3.3 mm (10 Fr). As a result, a covered metallic stent would not fit into such a working channel and therefore could not be used to treat a stricture located in the duodenal region. Accordingly, the benefits of a covered metal stent may not be realized in the duodenal region.

There is an unmet need for overcoming the space limitations currently associated with delivering covered metal stents to various regions of the body. Although the inventions described below may be useful for delivering a covered metal stent, the claimed inventions may also solve other problems as well.

It may be noted that devices have been proposed for use in percutaneous transluminal coronary angioplasty (PTCA) or percutaneous transluminal angioplasty (PTA) procedures that first deploy a plaque-trapping device including a tubular net within an area of plaque to be treated, and then deliver and place a primary stent into the area of treatment.

Specifically, WO01/82831 teaches a delivery catheter for delivering a plaque-trapping device. The delivery catheter includes an inner member which has a first mounting region and a second mounting region formed therein. The first mounting region is utilized for mounting a primary stent which is to be deployed within the area of treatment in the patient's vasculature. The second mounting region is designed for mounting the plaque-trapping device. A restraining sheath extends over the inner member, along with the stent and plaque-trapping device. This restraining sheath is designed to move axially in a longitudinal direction to first deploy the plaque-trapping device within the area of plaque to be treated and then to deliver and place the primary stent into the area of treatment for performance of the interventional procedure.

The document teaches that the plaque-trapping device includes a tubular net and may have respective expandable end rings attached to its proximal and distal ends. The plaque-trapping device also includes number of longitudinal struts, each of which is attached to the expandable members.

### SUMMARY

The invention may include any of the following aspects in various combinations and may also include any other aspect described below in the written description or in the attached drawings.

In a first aspect, a delivery system is provided comprising an outer sheath, a sleeve member disposed within the outer sheath, a metallic stent disposed within the outer sheath proximal of the sleeve member, wherein the sleeve member is configured to be deployed within a body lumen and further wherein the metallic stent is configured to be deployed within the sleeve member.

In a second aspect, a two-stage delivery method for deploying a dilation device is provided. The dilation device comprises a sleeve member and a metallic stent, the sleeve member disposed within an outer sheath, a metallic stent disposed within the outer sheath proximal of the sleeve member and between a pusher member and the outer sheath, the metallic stent disposed on the pusher member. The dilation device is advanced to a target site. The outer sheath is proximally retracted relative to the pusher member so as to expose the sleeve member. The sleeve member is expanded from a folded configuration to a radially expanded configuration
within the target site. The next step involves simultaneously advancing the second metal stent, the pusher member and the outer sheath so as to position the second metal stent within the sleeve member. The outer sheath is retracted relative to the pusher member to expose and deploy the metallic stent within the sleeve member.

### BRIEF DESCRIPTION OF SEVERAL VIEWS OF THE DRAWINGS

The embodiments are described with reference to the drawings in which like elements are referred to by like numerals. The relationship and functioning of the various elements of the embodiments are better understood by the following detailed description. However, the embodiments as described below are by way of example only, and the invention is not limited to the embodiments illustrated in the drawings. It should also be understood that the drawings are not to scale and in certain instances details have been omitted, which are not necessary for an understanding of the embodiments, such as conventional details of fabrication and assembly.

The invention may be more fully understood by reading the following description in conjunction with the drawings, in which:
Figure 1 shows a cross sectional view of an introducer system having a sleeve member and a metallic stent preloaded within the introducer system;
Figure 2 shows a cross-sectional view of Figure 1 along A-A;
Figure 3 shows a cross sectional view of the sleeve member in an expanded configuration;
Figure 4 is a blown-up view of Figure 1 showing a tapered distal end of the outer sheath in which the outer sheath having weakened longitudinal lines;
Figure 5 shows the preloaded delivery system at a target site within a body lumen;
Figure 6 shows the outer sheath having been proximally pulled back to expose the sleeve member with the sleeve member expanding within the body lumen;
Figure 7 shows the delivery system with metallic stent loaded on the pusher member being advanced distally and positioned within the sleeve member;
Figure 8 shows the outer sheath retracted within the sleeve member so as to cause the metallic stent to expand within the sleeve member;
Figure 9 shows the wire guide and device being removed from the target site, thereby leaving the metallic stent deployed and expanded within the interior space of the sleeve member to form an implanted covered stent assembly;
Figure 10 shows an alternative configuration of a sleeve member loaded within an outer sheath in a pleated configuration; and
Figure 11 shows an alternative configuration of a sleeve member loaded within an outer sheath and having a fold extending along its longitudinal axis.

### DETAILED DESCRIPTION

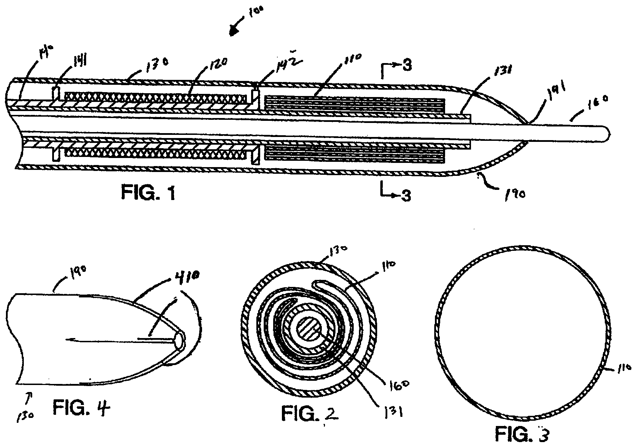

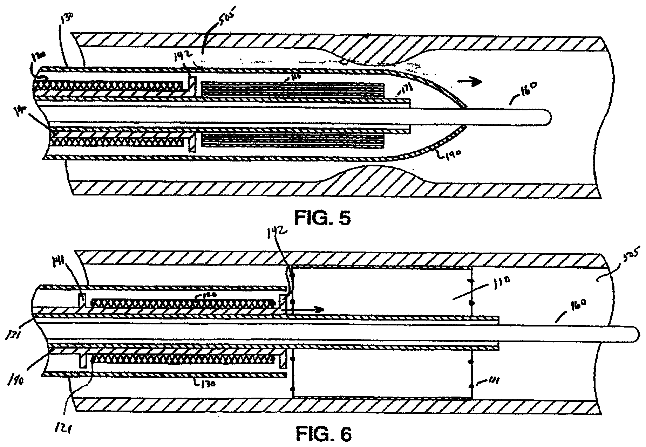

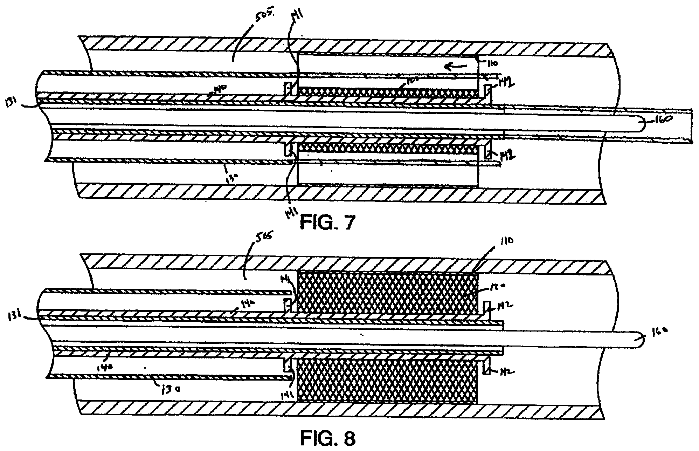

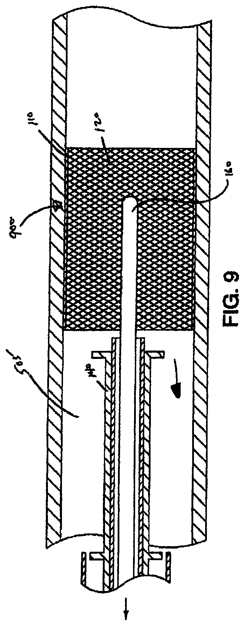

Figure 1 illustrates an exemplary delivery system 100 comprising a metallic stent and a sleeve member preloaded within a single introducer. The delivery system 100 comprises a sleeve member 110 and a spaced apart metallic stent 120 each disposed between an outer sheath 130 and an inner catheter 131. The metallic stent 120 is disposed proximally of the sleeve member 110. Both structures 110 and 120 are radially constrained within the outer sheath 130 in which each stent 110 and 120 have substantially identical radially compressed diameters. The preloaded delivery system 100 is designed such that the sleeve member 110 is deployed and self-expands within a body lumen 505 (Figure 6). Thereafter, the metallic stent 120 is subsequently deployed within the sleeve member 110 and expands within the sleeve member 110 (Figures 8 and 9). The metallic stent 120 presses radially outwards against the inner surfaces of the sleeve member 110 so as to further outwardly push the sleeve member against the walls of the body lumen 505 (Figures 8 and 9).

The metallic stent 120 is shown in Figure 1 to be preloaded between pusher member 140 and the outer sheath 130. The metallic stent 120 is axially disposed on a pusher member 140. The pusher member 140 comprises a first shoulder 141 and a second shoulder 142. The first shoulder 141 is shown in Figure 1 to be located at a proximal end of the metallic stent 120. The first ring-like 141 structure is in engagement with a proximal end of the metallic stent 120. The second shoulder 142 is shown in Figure 1 to be located at a distal end of the pusher member 140. The second shoulder 142 is in engagement with a distal end of the metallic stent 120. The proximal and distal shoulders 141 and 142 engage the respective proximal and distal ends of the metallic stent 120. The distal shoulder 142 prevents the sleeve member 110 from frictionally engaging with the outer sheath 130 during proximal movement of the outer sheath 130, thereby preventing proximal movement of the sleeve member 110 back into the delivery system 100. The proximal shoulder 141 prevents the metallic stent 120 from frictionally engaging with the outer sheath 130 during proximal movement of the sheath 130, thereby preventing proximal movement of the metallic stent 120 back into the delivery system 100. The shoulders 141 and 142 may also act as restraining members to prevent substantial longitudinal movement of the metallic stent 120 when the constraining outer sheath 130 is withdrawn. The preferred wall thickness of the metallic stent is from about .12 mm to about .3 mm.

The sleeve member 110 is shown spaced apart distally of the metallic stent 120. The sleeve member 110 is designed to sufficiently expand (Figure 3) from its compressed, folded configuration such that metallic stent 120 can be subsequently expanded therewithin.

The sleeve member 110 is collapsed and folded on itself within the outer sheath 130, as shown Figure 2, which shows a cross-sectional view of Figure 1 along A-A. The sleeve member 110 is compressed and folded between the outer sheath 130 and the inner catheter 131. The sleeve member 110 may be readily deformed, repeatedly, if necessary, prior to loading into the outer sheath 130. One way to configure the sleeve member 110 as shown in Figure 2 is to flatten the sleeve member 110 in the longitudinal direction and subsequently fold it in the longitudinal direction. As Figure 2 shows, the sleeve member 110 is compressed and folded about a wire guide 160, which extends within the inner sheath 131 through the structures 110 and 120. Although the inner catheter 131 and pusher member 140 are shown as two distinct structures, the inner catheter 131 and pusher member 140 may be integrated as a single structure.

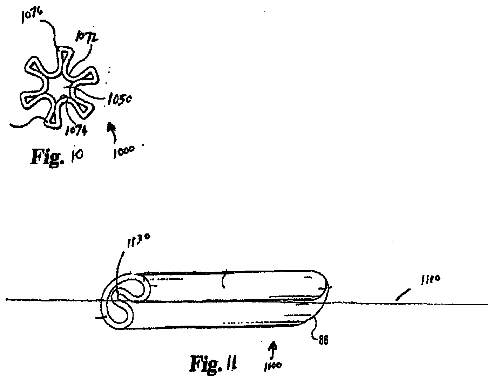

Alternatively, the sleeve member may be manufactured as a sleeve member 1000 having a plurality of folds 1072 extending along its longitudinal axis, as shown in Figure 10. Figure 10 shows a sleeve member 1000 loaded within an outer sheath (not shown) in a pleated configuration. The sleeve member 1000 resembles a circular pleated sheet or an accordion. The sleeve member 1000 has an interior surface 1074 that can collapse back on itself.

Alternatively, the sleeve member 1100 may comprise a pliable sleeve-like structure that is readily deformable as shown in Figure 11. The stent 1100 has a single fold 1130 extending along its longitudinal axis 1110. Although a single fold 1130 is shown in Figure 11, the stent 1100 can have two or more folds extending along its longitudinal axis 1110.

Examples of suitable polymeric materials to configure the above-described sleeve members include polyurethane or silicone. The sleeve members may have a wall thickness ranging from about .2 mm to about 1 mm. A relatively thinner wall thickness of the sleeve member 110 enables a greater extent to which it can be folded and compressed within the outer sheath 130 (Figure 2) during the loading of the sleeve member within the delivery system 100. A relatively thicker wall thickness enables the sleeve member to exert a greater force against a body lumen when expanded. One of ordinary skill in the art would be able to balance these competing design considerations to produce a sleeve member 110 that can sufficiently fold into a compressed profile during delivery (Figure 2) and yet sufficiently expand upon deployment so as to allow subsequent insertion of the metallic stent 120 within the interior region of the sleeve member 110 (Figures 5-9).

Referring to Figures 1 and 2, the outer sheath 130 is shown disposed over the metallic stent 120 and the sleeve member 110. The portion of the outer sheath 120 extending beyond the distal end of the polymeric sheath 110 may be tapered as shown in Figure 1. The tapered portion 190 creates an atraumatic end during delivery and deployment of the structures 110 and 120, as well as a streamlined delivery profile. The tapered portion 190 may have a sufficient distal opening 191 for a wire guide 160 to extend therethrough, as shown in Figure 1. It should be understood that the preloaded delivery system 100 can deliver and deploy the structures 110 and 120 with or without the wire guide 160.

Figure 4 shows that the tapered portion 190 may contain weakened longitudinal lines 410 to facilitate proximal retraction of the outer sheath 130 and deployment of the sleeve member 110. The longitudinal lines 410 may comprise a series of grooves or the like spaced about the tapered portion 190. Such grooves preweaken the tapered portion 190 of the outer sheath 130 so as to allow longitudinal tearing of the outer sheath 130 along the longitudinal lines 410. Alternatively, the material of the outer sheath 130 at the tapered portion 190 may be longitudinally molecularly oriented according to the method disclosed in U.S. Patent No. 4,306,562.

Having described the structure of the preloaded delivery system 100, a method of deploying the sleeve member 110 and the metallic stent 120 using the preloaded delivery system 100 will now be described. Generally speaking, the deployment occurs in two stages, the first stage involving deployment of the sleeve member 110 and the second stage involving deployment of the metallic stent 120 within the deployed sleeve member 110. Figure 5 shows the preloaded delivery system 100 of Figure 1 being distally advanced (as indicated by the distal arrow adjacent to the distal end of delivery system 100) over a wire guide 160 to a target site within a body lumen 505 where the structures 110 and 120 will be deployed. The proximal and distal shoulders 141 and 142 engage the metallic stent 120 so as to maintain separation of the metallic stent 120 from the sleeve member 110. The diameters of the loaded and compressed polymeric and metallic structures 110 and 120 are substantially equal.

Having delivered the delivery system 100 to the target site, the first stage of deployment can now occur in which the sleeve member 110 is expanded within the body lumen 505. The outer sheath 130 is proximally retracted relative to the inner member 131 and the pusher member 140. Figure 6 shows the outer sheath 130 having been partially pulled back in the proximal direction so as to completely expose the sleeve member 110. To facilitate deployment of the sleeve member 110, the proximal retraction of the outer sheath 130 also causes the tapered distal portion 190 to longitudinally tear along the weakened longitudinal lines 410 (Figure 4). Upon retraction of the outer sheath 130 as shown in Figure 6, the sleeve member 110 unfolds from its compressed configuration about the inner catheter 131 (Figure 2) and self-expands within the body lumen 505 (Figures 3 and 6). The distal shoulders 142 may function to prevent the sleeve member 110 from frictionally engaging with the outer sheath 130 and proximally moving with the outer sheath 130 into the delivery system 100. As will be explained in greater detail below, the sleeve member 110 must be sufficiently expanded to allow subsequent introduction of the metallic stent 120 therein. The distal end of the outer sheath 130 remains positioned at about the distal shoulders 142 so as to maintain constrainment of the metallic stent 120 within the outer sheath 130.

Having deployed the sleeve member 110 at the target site of the body lumen 505, the metallic stent 120 may now be advanced and positioned within the deployed sleeve member 110. Referring to Figure 6, the delivery system 100 is advanced forward into the interior space of the sleeve member 110 as indicated by the distal arrow adjacent to the distal end of delivery system 100. In particular, the outer sheath 130, inner member 131, and pusher member 140 with metallic stent 120 loaded thereon are simultaneously advanced distally along the inner catheter 131 inside of the sleeve member 110 so as to position the delivery system 100 as shown in Figure 7. Radiopaque markers 111 (Figure 6) may be circumferentially positioned along the proximal and distal ends of the sleeve member 110 and radiopaque markers 121 (Figure 6) may be positioned along the distal ends of metallic stent 120 so as to assist in aligning the proximal and distal ends of the metallic stent 120 within the sleeve member 110 by visually monitoring the relative location of the radiopaque proximal and distal ends of structures 110 and 120 (Figure 7). Alternatively, the entire sleeve member 110 may be made of elastic materials mixed and/or impregnated with radiopaque additives.

Having advanced and positioned the delivery system 100 within the sleeve member 110, the second stage of deployment can now occur in which the metallic stent 120 is expanded within the sleeve member 110. The outer sheath 130 is proximally retracted, as indicated by the distal arrow in Figure 7. As the outer sheath 130 retracts, the metallic stent 120 begins to self-expand. When the distal end of the outer sheath 130 has passed the proximal end of the metallic stent 120, the metallic stent will be fully expanded within the sleeve member 110. Figure 8 shows the metallic stent 120 having expanded within the sleeve member 110 and aligned with the sleeve member 110. The metallic stent 120 self-expands and exerts a radial force against the inner surfaces of the sleeve member 110, thereby radially pushing out the sleeve member 110 such that the outer surfaces of the sleeve member 110 engage with the stricture (Figure 8).

At this juncture, the delivery system 100 may be withdrawn from the target site of the body lumen 505 (Figure 9). The pusher member 140 is proximally retracted from the interior of the metallic stent 120 and returned back to within the outer sheath 130. The delivery system 100 may then be withdrawn along with the wire guide 160. Figure 9 shows the wire guide 160, pusher member 140, and outer sheath 130 being retracted in the proximal direction (as indicated by the arrow) from the target site of the body lumen 505.

Figure 9 also shows the implanted stent assembly 900. The sleeve member 110 has an expanded diameter less than that of the metallic stent 120 when each of the structures 110 and 120 is expanded individually. When the metallic stent 120 is deployed within the sleeve member 110, the metallic stent 120 is configured to expand to a diameter greater than that of the sleeve member 110 so as to exert an outward radial force against the sleeve member 110. The sleeve member 110 is pushed outwardly such that it engages the stricture at the target site of the body lumen 505. In this example, when each of the structures 110 and 120 is individually deployed, the expanded diameter of the sleeve member is about 9 mm, and the expanded diameter of the metallic stent is about 10 mm. Additionally, the metallic stent 120 may comprise a greater longitudinal length than the sleeve member 110 so as create widened ends that fixate the stent assembly 900 at the target site without migrating. The outer sleeve member 110 may be made from a SMP (shape memory plastic, which will be discussed below) which is porous so as to impregnate the pores with various bioactives.

In an alternative embodiment, the sleeve member 110 may be formed from a shape memory plastic (SMP) which can change from a temporary deformed shape back to its memorized or remembered shape in response to one or more certain stimuli. A sleeve member 110 formed from a SMP may be cast as a thin-wall tube, woven as a sleeve of polymeric fibers, or comprise a combination of a thin-walled tube and a woven sleeve. Once the stored remembered state has been produced by conventional methods (e.g., extrusion), the SMP is molded into a second, temporary deformed form by heating, deformation, and finally, cooling. As a result, the SMP stent may revert back or self-expand from its temporary deformed shape (i.e. the folded and compressed configuration of Figure 2) to its remembered or memorized shape (i.e., the unfolded and uncompressed configuration of Figure 3) within seconds at a predetermined switching temperature. The ability of the SMP stent to change shape is based on its built-in elastic memory that has been imparted to it during fabrication. The SMP stent reverts back to its remembered state by subjecting the sleeve member to one or more stimuli, including adsorption of heat by the polymer, adsorption of liquid by the polymer, and a change in pH in the liquid in contact with the polymer.

An example of the procedure for imparting shape memory characteristics to a sleeve member is as follows. The temperatures and dimensions to be described below are for illustration purposes only and not intended to be limiting in any way. After the sleeve member is formed by conventional techniques known in the art (e.g., tube spinning, extrusion) the sleeve member is stretched or deformed at an elevated temperature (e.g., about 40°C) and held in the stretched state until cooled to about 20°C so as to fixate the stent in the stretched (compressed) state. The stent remains fixated in the stretched state as long as the temperature it is subjected to remains below about 40°C. By way of example, the prestretched diameter (remembered state) can be about 5.0 mm, whereas the stretched (deformed state) can be reduced to an outside diameter of about 3.0 mm. Thereafter, the sleeve member can self-expand or revert back to its original remembered state by heating the same to above the switching temperature of 40°C, which is also commonly referred to as the glass transition temperature Tg. Generally speaking, heating the polymeric material above its glass transition temperature Tg enables the polymeric material to soften and be reshaped to another configuration (i.e., the temporary deformed state), and cooling of the material below the temperature Tg causes the material to stiffen and retain the reshaped configuration until the material is reheated to above the temperature Tg causing the material to return to its original remembered shape.

The exact temperature regime under which the stent can be fixedly deformed into the temporary shape varies depending on numerous factors, including the type of polymer and the structure of the polymer (e.g., the absence or presence of cross-linking agents). For example, one component, oligo(e-caprolactone) dimethacrylate, furnishes the crystallizable "switching" segment that determines both the temporary deformed shape and permanent remembered shape of the polymer. By varying the amount of the comonomer, n-butyl acrylate, in the polymer network, the cross-link density can be adjusted. In this way, the mechanical strength and transition temperature of the polymers can be tailored over a wide range.

Any suitable polymeric materials known in the art may be used for the SMP stent including poly(methylmethacrylate) (PMMA) based materials, poly(vinylchloride), polyurethane-based materials, and silicone, poly-L-lactic acid, polycarbonate, polyethylene terephthalate or engineering plastics such as thermotropic liquid crystal polymers (LCPs)); biocompatible polymeric materials (e.g., cellulose acetate, cellulose nitrate, silicone, polyethylene terephthalate, polyurethane, polyamide, polyester, polyorthoester, polyanhydride, polyether sulfone, polycarbonate, polypropylene, high molecular weight polyethylene or polytetrafluoroethylene); degradable or biodegradable polymers, plastics, natural (e.g., animal, plant or microbial) or recombinant material (e.g., polylactic acid, polyglycolic acid, polyanhydride, polycaprolactone, polyhydroxybutyrate valerate, polydepsipeptides, nylon copolymides, conventional poly(amino acid) synthetic polymers, pseudo-poly(amino acids) or aliphatic polyesters (e.g., polyglycolic acid (PGA), polylactic acid (PLA), polyalkylene succinates, polyhydroxybutyrate (PHB), polybutylene diglycolate, poly epsilon-caprolactone (PCL), polydihydropyrans, polyphosphazenes, polyorthoesters, polycyanoacrylates, polyanhydrides, polyketals, polyacetals, poly(.alpha.-hydroxy-esters), poly(carbonates), poly(imino-carbonates), poly(.beta.-hydroxy-esters) or polypeptides)); polyethylene terephthalate (e.g., dacron or mylar); expanded fluoropolymers (e.g., polytetrafluoroethylene (PTFE)); fluorinated ethylene propylene (FEP); copolymers of tetrafluoroethylene (TFE) and per fluoro(propyl vinyl ether) (PFA)); homopolymers of polychlorotrifluoroethylene (PCTFE) and copolymers with TFE; ethylene-chlorotrifluoroethylene (ECTFE); copolymers of ethylene-tetrafluoroethylene (ETFE); polyvinylidene fluoride (PVDF); polyvinyfluoride (PVF); polyaramids (e.g., kevlar); polyfluorocarbons including polytetrafluoroethylene with and without copolymerized hexafluoropropylene (e.g., teflon or goretex); expanded fluorocarbon polymers; polyglycolides; polylactides; polyglycerol sebacate; polyethylene oxide; polybutylene terepthalate; polydioxanones; proteoglycans; glycosaminoglycans; poly(alkylene oxalates); polyalkanotes; polyamides; polyaspartimic acid; polyglutarunic acid polymer; poly-p-diaxanone (e.g., PDS); polyphosphazene; polyurethane including porous or nonporous polyurethanes; poly(glycolide-trimethylene carbonate); terpolymer (copolymers of glycolide, lactide or dimethyltrimethylene carbonate); polyhydroxyalkanoates (PHA); polyhydroxybutyrate (PHB) or poly(hydroxybutyrate-co-valerate) (PHB-co-HV); poly(epsilon-caprolactone) (e.g., lactide or glycolide); poly(epsilon-caprolactone-dimethyltrimethylene carbonate); polyglycolic acid (PGA); poly-L and poly-D(lactic acid) (e.g., calcium phosphate glass); lactic acid/ethylene glycol copolymers; polyarylates (L-tyrosine-derived) or free acid polyarylates; polycarbonates (tyrosine or L-tyrosine-derived); poly(ester-amides); poly(propylene fumarate-co-ethylene glycol) copolymer (e.g., fumarate anhydrides); polyanhydride esters; polyanhydrides; polyorthoesters; prolastin or silk-elastin polymers (SELP); calcium phosphate (bioglass); compositions of PLA, PCL, PGA ester; polyphosphazenes; polyamino acids; polysaccharides; polyhydroxyalkanoate polymers; various plastic materials; teflon; nylon; block polymers or copolymers; Leica RM2165; Leica RM2155; organic fabrics; biologic agents (e.g., protein, extracellular matrix component, collagen, fibrin); small intestinal submucosa (SIS) (e.g., vacuum formed SIS); collagen or collagen matrices with growth modulators; aliginate; cellulose and ester; dextran; elastin; fibrin; gelatin; hyaluronic acid; hydroxyapatite; polypeptides; proteins; ceramics (e.g., silicon nitride, silicon carbide, zirconia or alumina); bioactive silica-based materials; carbon or carbon fiber; cotton; silk; spider silk; chitin; chitosan (NOCC or NOOC-G); urethanes; glass; silica; sapphire; composites; any mixture, blend, alloy, copolymer or combination of any of these; or various other materials not limited by these examples.

Some of the above-described SMP materials are also known to be biodegradable such that the outer deployed SMP stent will biodegrade over time, thereby leaving behind a metallic stent 120 without any tissue ingrowth. Because the metallic stent 120 does not have any tissue ingrowth, it can readily be removed when the outer SMP stent has biodegraded. Examples of such SMP materials which are also biodegradable include oligo(ε-caprolactone)diol and crystallisable oligo(p-dioxanone)diol; polysaccharides such as alginate, dextran, cellulose, collagen, and chemical derivatives thereof (substitutions, additions of chemical groups, for example, alkyl, alkylene, hydroxylations, oxidations, and other modifications routinely made by those skilled in the art), and proteins such as albumin, zein and copolymers and blends thereof, alone or in combination with synthetic polymers. The SMP biodegradable materials can degrade by hydrolysis, by exposure to water or enzymes under physiological conditions, by surface erosion, bulk erosion, or a combination thereof.

As can be seen, the above delivery system 100 preloaded with a sleeve member 110 and a metallic stent 120 provides a novel 2-stage delivery for deploying a covered stent assembly 900 at target strictures that would not otherwise be possible because of limited introducer space. Accordingly, many target strictures can now receive the benefit of a polymeric covering over a metal stent. The ability to use a smaller introducer also translates into a more manageable procedure for the physician as smaller sized introducers are easier to negotiate around bends of a body lumen compared to larger-sized introducers.

While preferred embodiments of the invention have been described, it should be understood that the invention is not so limited, and modifications may be made without departing from the invention. The scope of the invention is defined by the appended claims, and all devices that come within the meaning of the claims, either literally or by equivalence, are intended to be embraced therein. Furthermore, the advantages described above are not necessarily the only advantages of the invention, and it is not necessarily expected that all of the described advantages will be achieved with every embodiment of the invention.

## Claims

1. A delivery system (100), comprising:
an outer sheath (130); a polymeric sleeve member (110, 1000, 1100) disposed within the outer sheath;
a metallic stent (120) disposed within the outer sheath proximal and spaced apart of the polymeric sleeve member (110, 1000, 1100), wherein the polymeric sleeve member (110, 1000, 1100) is configured to be deployed within a body lumen;
wherein the polymeric sleeve member (110, 1000, 1100) is compressed and folded on itself within the outer sheath (130); and
further wherein the metallic stent (120) is configured to be deployed within the polymeric sleeve member (110, 1000, 1100) subsequent to deployment of the sleeve member.

2. The delivery system according to claim 1,wherein the sleeve member (110, 1000, 1100) comprises one or more folds (1072, 1130) extending along its longitudinal axis (1110) and preferably wherein the sleeve member is configured by being flattened and subsequently folded in the longitudinal direction.

3. The delivery system according to claim 1, wherein the metallic stent (120) is axially disposed on a pusher member (140), the pusher member (140) being disposed along an inner sheath (131) coaxially disposed within the outer sheath (130).

4. The delivery system according to claim 3, further comprising a guide wire extending through the polymeric sleeve member (110, 1000, 1100), the metallic stent (120), and at least a portion of the inner sheath (131).

5. The delivery system according to claim 1, wherein the polymeric sleeve member (110, 1000, 1100) is expandable from a delivery configuration to an expanded configuration having a larger diameter.

6. The delivery system according to claim 1, the polymeric sleeve member (110, 1000, 1100) comprising a first delivery diameter within the outer sheath (130) and the metallic stent (120) comprising a second delivery diameter within the outer sheath (130), wherein the first diameter and the second diameters are about equal.

7. The delivery system according to claim 3, the pusher member (140) further comprising a first shoulder (141) at a proximal end of the pusher member (140), the first shoulder being in engagement with a proximal end of the first metallic stent (120).

8. The delivery system of claim 7, wherein the first shoulder (141) is configured to engage the proximal end of the polymeric sleeve member (110, 1000, 1100) as the outer sheath (130) is withdrawn during deployment.

9. The delivery system according to claim 2, the pusher member (140) further comprising a second shoulder (142) at a distal end of the pusher member (140), the second shoulder being in engagement with a distal end of the metallic stent (120).

10. The delivery system of claim 9, wherein the second shoulder (142) is configured to engage the proximal end of the metallic stent (120) as the outer sheath (130) is withdrawn during deployment

11. The delivery system of claim 1, wherein the polymeric sleeve member (110, 1000, 1100) comprises a shape memory plastic.

12. The delivery system according to claim 1, wherein the polymeric sleeve member (110, 1000, 1100) comprises a radiopaque marker.

13. The delivery system according to claim 1, wherein the outer sheath (130) comprises a tapered distal end that is configured to separate upon proximal movement relative to the inner sheath (131).

14. The delivery system according to claim 3, wherein the pusher member (140) and the inner sheath (131) comprise an unitary construction.

15. A delivery system according to claim 1 configured and adapted for use in two-stage delivery method comprising the steps of:
(a) advancing the delivery system to a target site;
(b) proximally retracting the outer sheath (130) relative to the pusher member (140) so as to expose the polymeric sleeve member (110, 1000, 1100);
(c) expanding the polymeric sleeve member (110, 1000, 1100) from a folded configuration to a radially expanded configuration within the target site;
(d) simultaneously advancing the second metal stent, the pusher member (140) and the outer sheath (130)so as to position the second metal stent within the polymeric sleeve member (110, 1000, 1100); and
(e) retracting the outer sheath (130) relative to the pusher member (140) to expose and deploy the metallic stent within the polymeric sleeve member (110, 1000, 1100).

## Patentansprüche

1. Freisetzungssystem (100), umfassend:
eine Außenhülle (130); ein Polymer-Hülsenelement (110, 1000, 1100), das in der Außenhülle angeordnet ist;
einen Metallstent (120), der in der Außenhülle proximal und im Abstand vom Polymer-Hülsenelement (110, 1000, 1100) angeordnet ist, wobei das Polymer-Hülsenelement (110, 1000, 1100) zur Freisetzung in einem Körperlumen ausgelegt ist;
wobei das Polymer-Hülsenelement (110, 1000, 1100) in der Außenhülle (130) komprimiert und umgefaltet ist; und
wobei ferner der Metallstent (120) zur Freisetzung in dem Polymer-Hülsenelement (110, 1000, 1100) im Anschluss an die Freisetzung des Hülsenelements ausgelegt ist.

2. Freisetzungssystem nach Anspruch 1, wobei das Hülsenelement (110, 1000, 1100) eine oder mehrere Falten (1072, 1130) umfasst, die sich entlang seiner Längsachse (1110) erstrecken, und wobei vorzugsweise das Hülsenelement dadurch konfiguriert wird, dass es flachgedrückt wird und anschließend in der Längsrichtung gefaltet wird.

3. Freisetzungssystem nach Anspruch 1, wobei der Metallstent (120) axial auf einem Schieberelement (140) angeordnet ist, wobei das Schieberelement (140) entlang einer Innenhülle (131) angeordnet ist, die koaxial innerhalb der Außenhülle (130) angeordnet ist.

4. Freisetzungssystem nach Anspruch 3, ferner umfassend einen Führungsdraht, der sich durch das Polymer-Hülsenelement (110, 1000, 1100), den Metallstent (120) und zumindest einen Teil der Innenhülle (131) erstreckt.

5. Freisetzungssystem nach Anspruch 1 wobei das Polymer-Hülsenelement (110, 1000, 1100) aus einer Freisetzungskonfiguration in eine expandierte Konfiguration mit einem größeren Durchmesser expandierbar ist.

6. Freisetzungssystem nach Anspruch 1, wobei das Polymer-Hülsenelement (110, 1000, 1100) einen ersten Freisetzungsdurchmesser innerhalb der Außenhülle (130) umfasst und der Metallstent (120) einen zweiten Freisetzungsdurchmesser innerhalb der Außenhülle (130) umfasst, wobei der erste Durchmesser und der zweite Durchmesser ungefähr identisch sind.

7. Freisetzungssystem nach Anspruch 3, wobei das Schieberelement (140) ferner eine erste Schulter (141) an einem proximalen Ende des Schieberelements (140) umfasst, wobei die erste Schulter mit einem proximalen Ende des ersten Metallstents (120) in Eingriff steht.

8. Freisetzungssystem nach Anspruch 7, wobei die erste Schulter (141) dazu ausgelegt ist, das proximale Ende des Polymer-Hülsenelements (110, 1000, 1100) in Eingriff zu nehmen, wenn die Außenhülle (130) während der Freisetzung zurückgezogen wird.

9. Freisetzungssystem nach Anspruch 2, wobei das Schieberelement (140) ferner eine zweite Schulter (142) an einem distalen Ende des Schieberelements (140) umfasst, wobei die zweite Schulter mit einem distalen Ende des ersten Metallstents (120) in Eingriff steht.

10. Freisetzungssystem nach Anspruch 9, wobei die zweite Schulter (142) dazu ausgelegt ist, das proximale Ende des Metallstents (120) in Eingriff zu nehmen, wenn die Außenhülle (130) während der Freisetzung zurückgezogen wird.

11. Freisetzungssystem nach Anspruch 1, wobei das Polymer-Hülsenelement (110, 1000, 1100) einen Formgedächtniskunststoff umfasst.

12. Freisetzungssystem nach Anspruch 1, wobei das Polymer-Hülsenelement (110, 1000, 1100) einen röntgenopaken Marker umfasst.

13. Freisetzungssystem nach Anspruch 1, wobei die Außenhülle (130) ein verjüngtes distales Ende umfasst, das dazu ausgelegt ist, sich bei einer proximalen Bewegung bezüglich der Innenhülle (131) zu teilen.

14. Freisetzungssystem nach Anspruch 3, wobei das Schieberelement (140) und die Innenhülle (131) eine einheitliche Konstruktion umfassen.

15. Freisetzungssystem nach Anspruch 1, das zur Verwendung in einem zweistufigen Freisetzungsverfahren ausgelegt und angepasst ist, umfassend die folgenden Schritte:
(a) Vorschieben des Freisetzungssystems zu einem Zielort;
(b) proximales Zurückziehen der Außenhülle (130) bezüglich des Schieberelements (140), um das Polymer-Hülsenelement (110, 1000, 1100) freizulegen;
(c) Expandieren des Polymer-Hülsenelements (110, 1000, 1100) aus einer gefalteten Konfiguration in eine radial expandierte Konfiguration innerhalb des Zielorts;
(d) gleichzeitiges Vorschieben des zweiten Metallstents, des Schieberelements (140) und der Außenhülle (130), um den zweiten Metallstent innerhalb des Polymer-Hülsenelements (110, 1000, 1100) zu platzieren; und
(e) Zurückziehen der Außenhülle (130) bezüglich des Schieberelements (140), um den Metallstent innerhalb des Polymer-Hülsenelements (110, 1000, 1100) freizulegen und freizusetzen.

## Revendications

1. Système de pose en place (100), comprenant :
une gaine externe (130) ; un élément de manchon polymère (110, 1000, 1100) disposé à l'intérieur de la gaine externe ;
une endoprothèse métallique (120) disposée à l'intérieur de la gaine externe proximale et espacée de l'élément de manchon polymère (110, 1000, 1100), l'élément de manchon polymère (110, 1000, 1100) étant conçu pour être déployé dans une lumière corporelle ;
dans lequel l'élément de manchon polymère (110, 1000, 1100) est comprimé et replié sur lui-même dans la gaine externe (130) ; et
en outre dans lequel l'endoprothèse métallique (120) est conçue pour être déployée dans l'élément de manchon polymère (110, 1000, 1100) après le déploiement de l'élément de manchon.

2. Système de pose en place selon la revendication 1, dans lequel l'élément de manchon (110, 1000, 1100) comprend un ou plusieurs plis (1072, 1130) s'étendant le long de son axe longitudinal (1110) et de préférence dans lequel l'élément de manchon est conçu en étant aplati et par la suite replié dans la direction longitudinale.

3. Système de pose en place selon la revendication 1, dans lequel l'endoprothèse métallique (120) est disposée de manière axiale sur un élément poussoir (140), l'élément poussoir (140) étant disposé le long d'une gaine interne (131) disposée de façon coaxiale à l'intérieur de la gaine externe (130).

4. Système de pose en place selon la revendication 3, comprenant en outre un guide-fil s'étendant à travers l'élément de manchon polymère (110, 1000, 1100), l'endoprothèse métallique (120) et au moins une partie de la gaine interne (131).

5. Système de pose en place selon la revendication 1, dans lequel l'élément de manchon polymère (110, 1000, 1100) est déployable depuis une configuration de pose en place vers une configuration déployée présentant un diamètre plus grand.

6. Système de pose en place selon la revendication 1, l'élément de manchon polymère (110, 1000, 1100) comprenant un premier diamètre de pose en place dans la gaine externe (130) et l'endoprothèse métallique (120) comprenant un second diamètre de pose en place dans la gaine externe (130), dans lequel les premier et second diamètres sont pratiquement égaux.

7. Système de pose en place selon la revendication 3, l'élément poussoir (140) comprenant en outre un premier épaulement (141) au niveau d'une extrémité proximale de l'élément poussoir (140), le premier épaulement étant en prise avec une extrémité proximale de la première endoprothèse métallique (120).

8. Système de pose en place selon la revendication 7, dans lequel le premier épaulement (141) est conçu pour entrer en prise avec l'extrémité proximale de l'élément de manchon polymère (110, 1000, 1100) à mesure que la gaine externe (130) est retirée pendant le déploiement.

9. Système de pose en place selon la revendication 2, l'élément poussoir (140) comprenant en outre un second épaulement (142) au niveau d'une extrémité distale de l'élément poussoir (140), le second épaulement étant en prise avec une extrémité distale de l'endoprothèse métallique (120).

10. Système de pose en place selon la revendication 9, dans lequel le second épaulement (142) est conçu pour entrer en prise avec l'extrémité proximale de l'endoprothèse métallique (120) à mesure que la gaine externe (130) est retirée pendant le déploiement.

11. Système de pose en place selon la revendication 1, dans lequel l'élément de manchon polymère (110, 1000, 1100) comprend un plastique à mémoire de forme.

12. Système de pose en place selon la revendication 1, dans lequel l'élément de manchon polymère (110, 1000, 1100) comprend un marqueur radio-opaque.

13. Système de pose en place selon la revendication 1, dans lequel la gaine externe (130) comprend une extrémité distale conique qui est conçue pour se détacher lors d'un mouvement proximal par rapport à la gaine interne (131).

14. Système de pose en place selon la revendication 3, dans lequel l'élément poussoir (140) et la gaine interne (131) comprennent une construction monobloc.

15. Système de pose en place selon la revendication 1 conçu pour et adapté à une utilisation dans un procédé de pose en place à deux étapes comportant les étapes consistant à :
(a) faire avancer le système de pose en place vers un site cible ;
(b) rétracter de façon proximale la gaine externe (130) par rapport à l'élément poussoir (140) de façon à faire apparaître l'élément de manchon polymère (110, 1000, 1100) ;
(c) déployer l'élément de manchon polymère (110, 1000, 1100) depuis une configuration repliée vers une configuration déployée dans le sens radial dans le site cible ;
(d) faire simultanément avancer la seconde endoprothèse métallique, l'élément poussoir (140) et la gaine externe (130) de façon à positionner la seconde endoprothèse métallique dans l'élément de manchon polymère (110, 1000, 1100) ; et
(e) rétracter la gaine externe (130) par rapport à l'élément poussoir (140) de façon à faire apparaître et à déployer l'endoprothèse métallique dans l'élément de manchon polymère (110, 1000, 1100).
